# EUROPEAN PATENT SPECIFICATION

(11) **EP 1 949 877 B1**
(45) Date of publication and mention of the grant of the patent: **20.06.2012**
(21) Application number: 08100732.0
(22) Date of filing: 22.01.2008
(51) Int. Cl.: A61F 9/007, A61B 19/00

(54) **Thermally robust illuminator probe**
Hitzefeste Beleuchtungssonde
Sonde à illuminateur robuste thermiquement

(30) Priority: 23.01.2007 US 886140 P
(43) Date of publication of application: 30.07.2008
(73) Proprietor: Novartis AG, 4056 Basel (CH)
(72) Inventor: Smith, Ronald T., Irvine, CA 92618 (US); Auld, Jack R., Laguna Niguel, CA 92677 (US); Lin, Dean Y., Chino Hills, CA 91709 (US)
(74) Representative: Hanna, Peter William Derek

(56) References cited:
- WO-A-2006/088938
- WO-A-2007/005313
- WO-A-2007/053666
- WO-A-2007/133267
- US-A- 5 725 514
- US-A1- 2002 025 130
- US-A1- 2005 271 340

## Description

### TECHNICAL FIELD OF THE INVENTION

The present invention relates generally to illumination probes. More particularly, the present invention relates to illumination probes with distal fiber tips. Even more particularly, embodiments of the present invention relate to thermally robust illumination probe tips.

### BACKGROUND OF THE INVENTION

Optical fibers are useful in many illumination applications. For example, they can be used as light guides in medical and other applications where bright light needs to be shone on a surgical site or a target. Generally, light probes are made of plastic optical fibers due to their many desirable features. For example, plastic optical fibers can be very flexible, easy to form, have relatively high numeric aperture ("NA"), and are generally less expensive than glass fibers. Furthermore, they are easier to stretch and have taper shapes formed into them than glass fibers. Commercially available plastic optical fibers are flexible even at 20 gauge diameters, are easy to form proximal bells, and have as high as 0.63 NA. Additionally, the optical links, connectors, and installations associated with plastic optical fibers are relatively inexpensive compared to glass optical fibers. Glass optical fibers are stiff at 20 gauge diameters, difficult in belling the proximal end, and relatively more expensive to install and maintain.

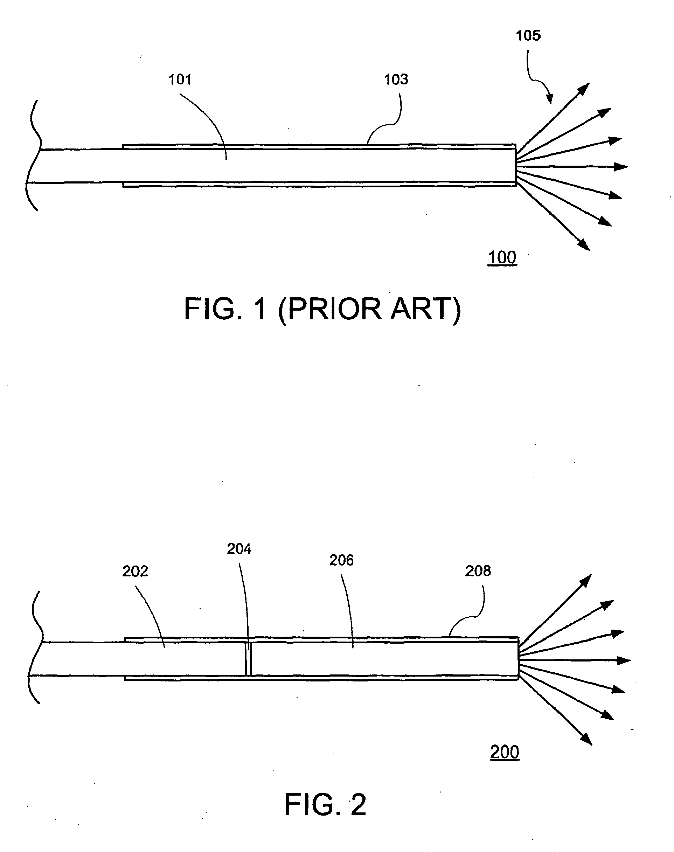

One drawback in making light probes out of plastic optical fibers is that they are vulnerable to deforming of the distal tips if they touch any absorptive material like blood or human tissue. As an example, Figure 1 shows the distal end of a simple endo-illumination probe 100 consisting of a plastic optical fiber 101 and a steel cannula 103. Plastic optical fiber 101 is a cylindrical dielectric waveguide comprised of a core surrounded by a cladding layer in a known manner. Plastic optical fiber 101 transmits light along its axis by the process of total internal reflection, an optical phenomenon known to those skilled in the art. The core of a plastic optical fiber 101 is highly optically transparent and is capable of transmitting very large amounts of white light luminous flux 105 without harming plastic optical fiber 101. This is possible because the absorbance of the optical fiber is very low. Therefore, large amounts of light are able to pass through the plastic fiber without heating it up to the softening or deformation point.

However, if any absorptive material, such as a drop of blood or a smear of human tissue, touches the end of probe 100, the following runaway cycle can quickly occur:
- The absorptive tissue absorbs a portion of visible light and heats up to a very high temperature (e.g., about 130°C or more);
- Being in physical contact with the optical fiber, the hot tissue causes the tip of the fiber to heat up;
- The temperature of the fiber tip exceeds the softening point of the fiber;
- The built-in linear compressive forces stored in the solid fiber are released, causing the tip of the fiber to recede and the diameter of the fiber tip to swell;
- As the tip recedes within the cannula, light emitted from the fiber now illuminates the distal tip of the cannula, causing it to get hot;
- The hot cannula causes the adjacent fiber to heat up past the softening temperature and eventually deform; and
- Very quickly, the fiber tip deformation renders the probe ineffective.

Due to this chain of reaction, when the light energy is delivered to an occluded fiber tip, the tip temperature may rise very quickly, causing the tip to deform and, in some cases, causing "mushrooming" of the tip. A fiber probe which no longer works is undesirable, but is not necessarily a safety hazard. However, if the light probe was inserted to a surgery site through a small incision, the mushrooming of the fiber tip could mean that the incision would need to be enlarged to fit the deformed probe tip. The current mitigation is to instruct the surgeon to limit the light output of the illumination source.

As an example, a chandelier probe is useful in illuminating a large area of a surgery site. In ophthalmic surgery, and in particular in vitreo-retinal surgery, it is desirable to view as large a portion of the retina as possible. Thus, a chandelier probe is sometimes inserted through a small incision hole in the sclera. If the plastic optical fiber in a chandelier probe were to deform into a rounded, swelled diameter ball, which it can do if absorptive contaminants touch the tip, the enlarged tip would be more difficult to pull back out through the incision hole. As a result, removing the probe might require the surgeon to enlarge the incision to prevent the wound from tearing. While this does not present a hazard to the patient, it is inconvenient to the surgeon and disrupts the normal flow of surgery.

A new solution is therefore needed to enjoy the advantages of plastic optic fibers without the problem of deforming distal tips. Embodiments of the invention disclosed herein can address this need and more.

WO 2006/088938 A (Alcon Inc.) describes a high throughput endo-illuminator having a distal tip optical fiber made of glass having a tapered section.

### SUMMARY OF THE INVENTION

Embodiments of the present invention provide a new solution to the problem of deforming distal tips of illumination probes. Specifically, embodiments of the present invention provide illumination probes having plastic optical fibers with thermally robust distal tips, in accordance with the claims which follow.

In the invention, the distal end of a plastic optical fiber is bonded to a high temperature distal part, which is short in length, is made of a high temperature material, and has a proper shape for guiding light in a desired application. In some embodiments, the high temperature distal part can be molded, machined or formed. In some embodiments, the high temperature distal part comprises one or more sections made of high temperature material(s) capable of transmitting light such as high temperature plastic tips. Other suitable high temperature materials are possible.

In embodiments of the invention, the distal end of a plastic optical fiber is bonded to a high temperature distal part using an optical adhesive. Additional components may be included to reinforce the optical bond and/or serve additional purpose(s). As an example, the distal end of a plastic optical fiber may be bonded to a high temperature distal part inside a steel cannula, a sleeve, an optical connector, or the like. As another example, the distal end of a plastic optical fiber may be bonded to a high temperature distal part inside and to a plastic hub made of one or more parts. Depending upon the configuration, a component such as a plastic hub may serve to anchor the illumination probe at a fixed position, to allow a fluid flow, or both. Other functions are also possible. In some embodiments, the illumination probe may incorporate an optical component at the high temperature distal end such as a particularly shaped sapphire ball which can function as a wide angle lens.

In some cases, it may be necessary to coat a portion of the high temperature distal part with a reflective coating to ensure that the light rays trapped within the part do not escape when the side of the part is in contact with anything but air (e.g., adhesive, cannula, sclera, etc.). Suitable coatings may include silver, aluminum, high reflectance dichroic coatings, etc.

Embodiments of the present invention provide many advantages over prior art. For example, unlike a convention distal fiber tip, a thermally robust illumination probe tip does not deform when some absorptive contaminants touch the tip, thus enabling users to enjoy the advantages and benefits of plastic optical fibers without having to worry about the problems and inconveniences caused by the deformation of distal tips.

### BRIEF DESCRIPTION OF THE DRAWINGS

A more complete understanding of the present invention and the advantages thereof may be acquired by referring to the following description, taken in conjunction with the accompanying drawings in which like reference numbers indicate like features and wherein:
FIGURE 1 is a diagrammatic representation of a simple endo-illumination probe consisting of a steel cannula and a plastic optical fiber with a conventional distal fiber tip that is prone to deformation when absorptive material touches the end of the probe;
FIGURE 2 is a diagrammatic representation of a thermally robust endo-illumination probe according to one embodiment of the present invention;
FIGURE 3 is a diagrammatic representation of a thermally robust chandelier probe according to another embodiment of the present invention;
FIGURE 4 is a diagrammatic representation of a thermally robust sapphire wide angle probe according to yet another embodiment of the present invention;
FIGURE 5 is a diagrammatic representation of a thermally robust illuminated infusion cannula according to one embodiment of the present invention;
FIGURE 6 is a diagrammatic representation of a proximally flared and distally tapered glass optical fiber suitable for implementing some embodiments of the invention such as in a thermally robust illuminated infusion cannula;
FIGURE 7 is a diagrammatic representation of a glass optical fiber having a protective sleeve enclosing the proximally flared portion of the glass optical fiber;
FIGURE 8 is a diagrammatic representation of a plastic optical fiber joining a glass optical fiber having a protective sleeve enclosing the proximally flared portion of the glass optical fiber inside an optical connector, forming an illumination probe with thermally robust tip, according to one embodiment of the present invention; and
FIGURE 9 is a diagrammatic representation of an omni-directionally reflective cannula suitable for implementing a thermally robust illumination probe according to one embodiment of the present invention.

### DETAILED DESCRIPTION

Preferred embodiments of the invention are illustrated in the FIGURES, like numerals being used to refer to like and corresponding parts of the various drawings.

The various embodiments of the present invention provide for illumination probes having plastic optical fibers with thermally robust distal tips. Embodiments of the invention can be applicable to plastic optical fibers of all gauges (e.g., 20, 23, 25, etc.). The current trend is toward 25 gauge and particularly 23 gauge - smaller diameter instructions that enable sutureless wounds in the sclera. The thermally robust illumination probe may be part of a surgical system (e.g., an ophthalmic illuminator device) useful in many medical procedures, such as in vitreo-retinal/posterior segment surgery. An exemplary ophthalmic illuminator may comprise a handpiece for delivering a beam of relatively incoherent light from a light source (e.g., a xenon light source, a halogen light source, or the like) through a fiber optic cable to a surgical area. Embodiments of the thermally robust illumination probes disclosed herein may be implemented for use with any appropriate handpieces, such as the Alcon-Grieshaber Revolution-DSP® handpiece sold by Alcon Laboratories, Inc., of Fort Worth, Texas. The distal end of the handpiece is coupled to a stem (cannula) or the like configured to house an illumination probe disclosed herein. In one embodiment, the thermally robust illumination probe is a disposable surgical item. It is contemplated and it will be realized by those skilled in the art that the scope of the present invention is not limited to ophthalmology, but may be applied generally to other areas of surgery where high throughput, higher gauge illumination may be required.

FIGURE 2 is a diagrammatic representation of a thermally robust endo-illumination probe 200 according to one embodiment of the present invention. Unlike probe 100 of FIGURE 1, stem 208 (e.g., a 25 gauge steel cannula) houses two different types of optical fibers: plastic optical fiber 202 and glass optical fiber 206, bonded together with optical adhesive 204. Optical adhesive 204 can be any index-matching optical-grade adhesive as will be known to those skilled in the art (e.g., Dymax 142-M optical adhesive, which rapidly cures upon exposure to ultraviolet or low wavelength visible light). Similarly, stem 208 can be stainless steel or a suitable biocompatible polymer (e.g., PEEK, polyimide, etc.) as will be known to those skilled in the art. Within this disclosure, stem 208 houses what is referred to as the distal optical fiber, the upstream end of which is optically coupled to the proximal optical fiber housed inside the optical cable connecting the handpiece and the light source. The proximal optical fiber is optically coupled to the light source. For best luminous flux performance, the thermally robust fiber should have a proximal diameter and NA equal to or greater than the distal diameter and NA of the plastic fiber. To avoid angular non-uniformities in the emitted beam, the proximal diameter of the thermally robust fiber should match the distal diameter of the plastic fiber as closely as possible, and the two fibers should be spatially aligned very well.

In one embodiment, plastic optical fiber 202 and glass optical fiber 206 are optically bonded together with optical adhesive 204 to form the distal optical fiber. Stem 208 can be attached to the distal optical fiber in any manner known to those skilled in the art. Glass optical fiber 206 provides the high temperature portion of illumination probe 200 that will not deform while emitting light during a surgery. Plastic optical fiber 202 provides a flexible optical conduit to receive light from a light source (e.g., through a proximal optical fiber and/or other optical couplings).

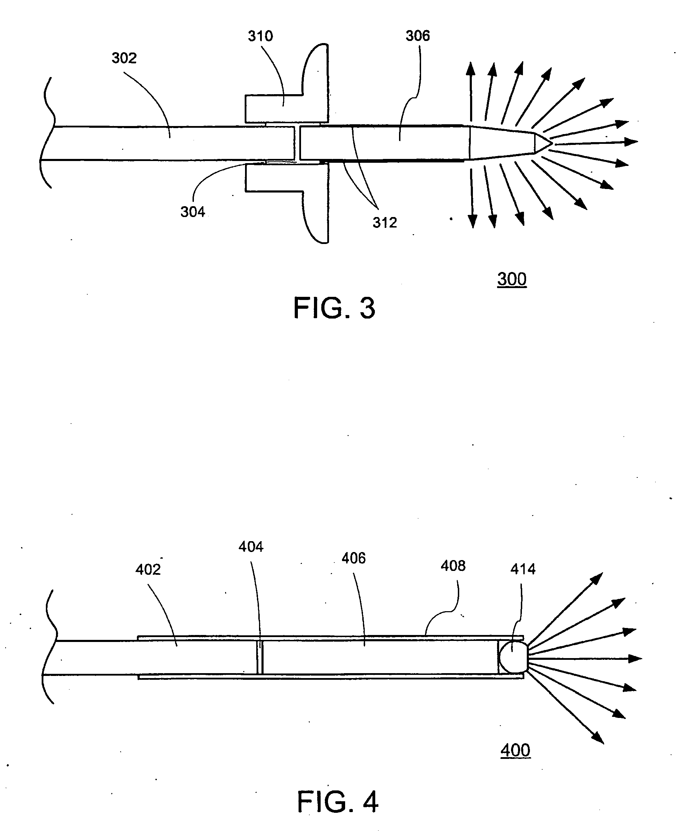

FIGURE 3 is a diagrammatic representation of an also disclosed thermally robust chandelier probe 300. In this example, plastic optical fiber 302 comes in and joins a molded or machined high temperature plastic tip 306 in a plastic hub 310. Hub 310 can be configured for anchoring probe 300 on the sclera so it will not move during the surgery. Hub 310 can provide a mechanical bond or otherwise support the adhesion between plastic optical fiber 302 and high temperature plastic tip 306. More specifically, optical adhesive 304 spills over from between plastic optical fiber 302 and high temperature plastic tip 306 into the gap between them and plastic hub 310 so there is adhesion between plastic optical fiber 302, hub 310 and high temperature plastic tip 306.

As illustrated in FIGURE 3, high temperature plastic tip 306 has a proximal end abutting plastic optical fiber 302 and a tapered section emitting light at its distal end. The tapered section can be fabricated by machining, diamond turning, casting, or injection molding. High temperature plastic tip 306 is a sculpted high temperature plastic rod that has no cladding to prevent light from escaping. Its NA is therefore dependent upon the refractive index of the tapered section and the refractive index of a surrounding medium. For example, if the tapered section is exposed to air, the NA of the tapered section will be essentially 1. This NA is much greater than the NA of the light beam passing through the tapered section; therefore, the transmittance of light through the tapered section can be as high as 100%. If the tapered section is likely to be exposed to liquid (e.g., saline solution from within an eye), the resultant NA is reduced but in some cases is still reasonably high. For example, if the high temperature plastic tip 306 material has a refractive index of 1.53 and the tip is immersed in saline solution which has a refractive index of approximately 1.36, the resultant NA is 0.70. However, if the high temperature plastic tip 306 touches either a high refractive index liquid, such as oil or optical adhesive, which typically has a refractive of 1.5 or higher, or an absorptive material such as hub 310 or the sclera, light will exit the plastic tip into the ambient medium and be lost. To prevent this from happening, a reflective coating 312 is applied on the outside of high temperature plastic tip 306 to confine the light within high temperature plastic tip 306 (i.e., so the light does not escape). Reflective coating 312 could be a reflective metal or dielectric coating. As one possibillity, silver coating is utilized. The length of the silver coating can be dependent upon the particular configuration of the light probe as well as the likelihood that the high temperature plastic tip is exposed to a surrounding medium other than the air or low refractive index liquid. While silver reflects about 98% of the light, it is not 100% reflective and therefore does not provide "total internal reflection". In this case, it might be desirable to minimize the length of the silver coating. On the other hand, the silver coating might be needed to protect the high temperature plastic rod from exposure to less desirable media (e.g., optical adhesive 304 spilled over from hub 310, fluid from a surgery site, etc.).

FIGURE 4 is a diagrammatic representation of a thermally robust sapphire wide angle probe 400 according to yet another embodiment of the present invention. Like probe 200 of FIGURE 2, stem 408 houses two different types of optical fibers: plastic optical fiber 402 and glass optical fiber 406, bonded together with optical adhesive 404. Optical adhesive 404 can be any index-matching optical-grade adhesive (e.g., Dymax 142-M optical adhesive). Similarly, stem 408 can be stainless steel or a suitable biocompatible polymer (e.g., PEEK, polyimide, etc.). In this example, stem 408 is configured to integrate sapphire ball 414 which functions as a wide angle lens. Stem 408 can be attached to plastic optical fiber 402 and glass optical fiber 406 in any manner known to those skilled in the art. Both glass optical fiber 406 and sapphire ball 414 are high temperature materials that will not deform while emitting light during a surgery.

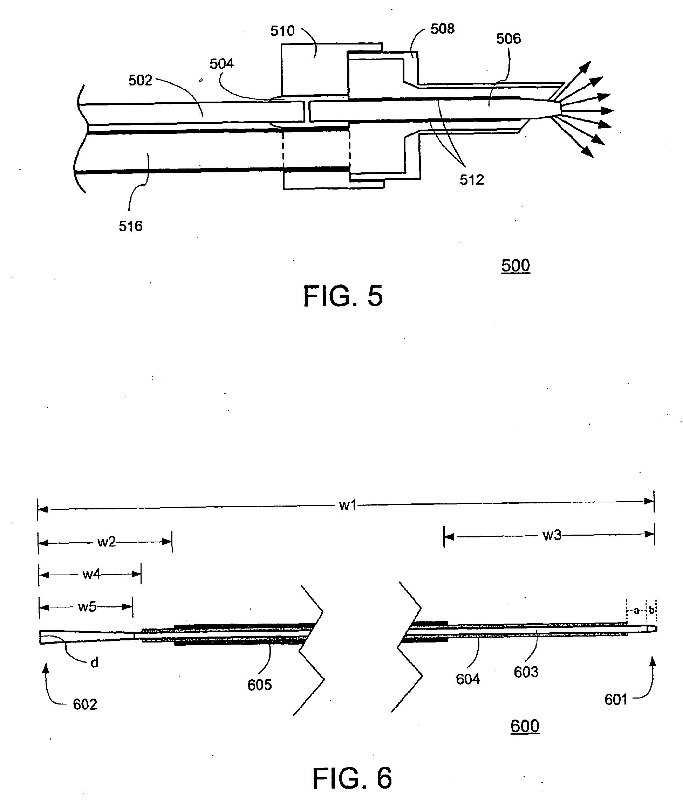

FIGURE 5 is a diagrammatic representation of an also disclosed thermally robust illuminated infusion cannula 500. Illuminated infusion cannula 500 can combine a fluid channel and an illumination probe to provide fluid flows, pressurization to the eye, and illumination, advantageously eliminating the need to have three separate probes inserted into a surgery site. In this example, flexible plastic hose 516 provides a channel for fluid flow and is attached to plastic cap or hub 510. Plastic optical fiber 502 and high temperature plastic rod 506 are bonded together inside hub 510 with optical adhesive 504. Optical adhesive 504 spills over from the gap between plastic optical fiber 502 and high temperature plastic rod 506 into the gap surrounding the joint to provide adhesion to hub 510 to position plastic optical fiber 502 properly along the cannula axis. However, it is not necessary for the fiber to be laterally centered relative to the cannula axis. Its optical performance will be unaffected even if it is along the side of the cannula touching the inside cannula wall. Plastic hose 516 and plastic optical fiber 502 may be housed in a protective sheath (not shown).

Hub 510 is particularly configured to precisely receive or otherwise tightly connect to trocar cannula 508. Trocar cannula 508 is configured to fit the downstream end of hub 510, to accommodate the fluid flow path from plastic hose 516, and to house high temperature plastic rod 506, which may or may not be co-axial with the fluid flow path. Like hub 310 of FIGURE 3, hub 510 and cannula 508 may be molded or machined out of plastic or other biocompatible material. Cannula 508 may function to anchor probe 500 at a fixed position (e.g., on the sclera). As one possibility, cannula 508 is comprised of two parts - a cylindrical steel or polyimide or PEEK cannula that is attached or bonded to another hub that is typically injection molded in plastic.

If high temperature plastic rod 506 is to be exposed to an absorptive medium such as the sclera or a high refractive index medium such as optical adhesive or oil, the portion that is exposed is coated with reflective coating 512 to prevent the light from escaping. The distal end of trocar cannula 508 may be sculpted for easy entry through an incision to a surgery site. The distal end of high temperature plastic rod 506 may be molded, machined or formed (e.g., laser thermal forming) to taper into a predetermined shape. One taper shape that efficiently spreads light over a wide range of angles while achieving high emission efficiencies is the compound parabola concentrator (CPC) - cone shape. Reflective coating 512 could be a reflective metal (e.g., silver) or dielectric (e.g. Teflon® or multilayer dielectric stack) coating.

FIGURE 6 is a diagrammatic representation of a proximally flared and distally tapered glass optical fiber 600 suitable for implementing some embodiments of the invention such as in a thermally robust illuminated infusion cannula. In one embodiment, fiber 600 is a FSU fiber available from Polymicro. It has a number of unique features which make it a suitable candidate as the high temperature distal part of a thermally robust illumination probe. For example:
- It has 475 µ +/- 13 µ Teflon® cladding 604 (9.0 µ +/- 3.0 µ in cladding thickness) with a very low refractive index (~1.30 - 1.33) that enables fiber 600 to achieve a very high numerical aperture (NA) of 0.66. NA is a measure of the acceptance angle of an optical fiber to propagating light.
- Its distal end 601 is tapered into a shape similar to a compound parabola concentrator (CPC) - cone shape. This can be done by way of laser thermal forming - using precision control of a high temperature laser to form distal end 601 into a pre-defined shape. In doing this step, the Teflon® cladding (developed by DuPont) bums off in the region ('a' + 'b') of laser forming. The non-tapered region 'a' should be as short as possible. As an example, the sculpted taper region 'b' can be 723.8 µ. As long as the bare exposed silica core 603 (457 µ +/- 10 µ) touches nothing other than air or saline solution, light will stay predominantly confined within fiber core 603.
- Its polished, planar proximal end 602 is linearly flared. Again, this can be done by way of laser thermal forming - using precision control of a high temperature laser to flare the fiber proximal end. In doing this step, the Teflon® cladding bums off in the region (w4) of laser forming. As an example, the flared region w5 can be about 3100 µ - 4500 µ with a diameter (d) of 737 µ +/- 10 µ at proximal end 602. The overall length of fiber 600 (w1) can be 12" +/- 0.25" with a portion covered in 552 µ +/- 30 µ silicone buffer 605. The uncovered sections (w2 and w3) can be 11.0 mm +/- 1.0 mm and 9.0 mm +/- 1.0 mm, respectively.

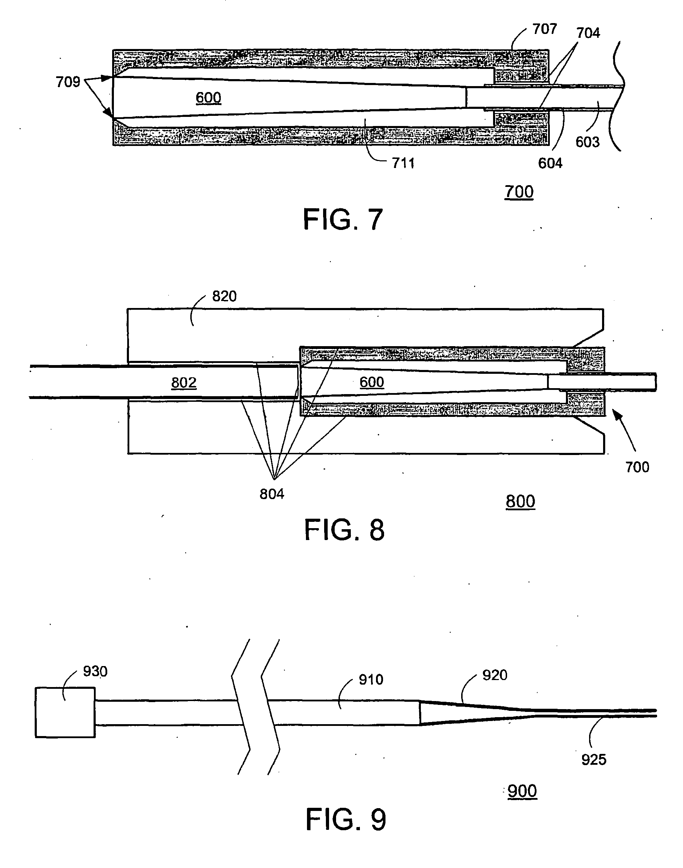

It is important that this bare exposed core 603 in the proximal flare region be exposed to nothing except air. FIGURE 7 is a diagrammatic representation of an optical component 700 comprising glass optical fiber 600 having a protective sleeve 707 enclosing the proximally flared portion of glass optical fiber 600 in air 711. Fiber 600 is bonded to protective sleeve 707, which may be made of glass, with adhesive 704. Protective glass sleeve 707 touches the bare proximally flared core 603 of fiber 600 only at points of contact 709, around the periphery of fiber 600 at the extreme proximal end thereof. This minimizes the leakage of light out of the flared core into the surrounding region.

FIGURE 8 is a diagrammatic representation of a fiber to fiber joint 800 comprising plastic optical fiber 802 joining optical component 700 and high temperature, high NA glass optical fiber 600 inside optical connector 820. According to one embodiment of the present invention, a high performance, thermally robust illumination fiber probe can be readily assembled by bonding the proximal end of glass optical fiber 600 to the distal end of plastic optical fiber 802 using optical adhesive 804 and connector 820, which is specially designed to accommodate optical element 700 having a protective sleeve enclosing the proximally flared portion of glass optical fiber 600.

FIGURE 9 is a diagrammatic representation of an omni-directionally reflective cannula suitable for implementing a thermally robust illumination probe according to one embodiment of the present invention. In this example, high throughput fiber probe 900 is connected to an ACMI connector 930 and comprises plastic optical fiber 910 optically bonded to a hollow, omni-directionally reflective cannula. The cannula has a hollow air core surrounded by a "cladding" that is a cylindrical coating with special properties.

Conventional coatings consist of (1) dielectric coating stacks, which have high reflectivity and little absorption but strong wavelength and angular selectivity, and (2) metal coatings which are omnidirectional reflectors with absorption losses and less than 100% reflectance. The special coatings described herein are like dielectric coatings, but consist of dielectric materials whose refractive indices are so different that the resultant coating combines the best of dielectric stacks and metallic coatings -- omnidirectional ultra-high reflectance over broad spectral and angular bandwidths. Essentially, a one-dimensional bandgap is created in the coating that prevents photons from entering the coating structure. Since they cannot enter the coating structure, they cannot transmit or absorb and therefore have no choice but to reflect.

The technology is proven for infrared wavelengths and if adapted to work in the visible wavelength band could yield potentially 100% reflectance within the hollow cannula. Thus, tapered hollow core fiber 920 and straight hollow core fiber 925 could potentially have fiber NAs as high as 1.0 (i.e., high transmittance for off-axis rays as high as 90 degrees off-axis) across the visible spectrum compared to the highest currently commercially available fiber NA of 0.63 (the Toray fiber).

In practice, there could be some attenuation losses, possibly up to about 0.65 dB/m. For a 100" long (2.54 meter) fiber this means that only 68% of the light is transmitted. However, 68% of a 1.0 NA beam provides significantly more light than 95% of a 0.63 NA beam. Furthermore, the specialty fiber would probably only be a short several-inch length used on the extreme distal end, so the resultant attenuating losses would be small.

If such a specialty fiber could be tapered, then a high throughput design as shown in FIGURE 9 is a possibility. More specifically, the 0.63 NA Toray fiber 910 would efficiently transport light from a fiber illuminator (e.g., Alcon's Accurus xenon illuminator) to the distal end. At that point, the tapered hollow-core fiber 920 would force the beam into a narrower diameter, causing the beam angular width (i.e., beam NA) to increase. For a fiber NA of 1, the beam after the tapered section will be transported efficiently through the narrow diameter straight section 925 of fiber 900. Note that filling the hollow core fiber with BSS or oil would affect its luminous flux propagation properties. If this specialty fiber could not be tapered, then a tapered glass or plastic rod (perhaps with silver coating on the side surface to confine the light within the taper) would be needed to couple light from the plastic proximal fiber to the hollow core distal cannula.

While the present invention has been described with reference to particular embodiments, it should be understood that the embodiments are illustrative and that the scope of the invention is not limited to these embodiments. Many variations, modifications, additions and improvements to the embodiments described above are possible. It is contemplated that these variations, modifications, additions and improvements fall within the scope of the invention as detailed in the following claims.

## Claims

1. A thermally robust illumination probe (200,300,400,500) comprising:
a plastic optical fiber (202,302,402,502);
a high temperature distal part (206,306,406,506,600); and
a housing (208,310,408,508);
wherein the plastic optical fiber is optically coupled to the high temperature distal part for transmitting light through the high temperature distal part, wherein the plastic optical fiber is bonded to the high temperature distal part together inside the housing,
wherein the high temperature distal part is a glass optical fiber having a cladding (604) and a core (603) adapted to enable the fiber to achieve a numerical aperture of 0.66, **characterized in that** the core is exposed at a distal end (601) and the distal end (601) is tapered into a compound parabola concentrator - cone shape.

2. The thermally robust illumination probe of claim 1, wherein the housing is a cannula (508) made of stainless steel or a biocompatible material.

3. The thermally robust illumination probe of claim 1, wherein the high temperature distal part (600) is made out of one or more high temperature materials.

4. The thermally robust illumination probe of claim 3, wherein the high temperature distal part (600) has a sculpted distal end (601).

5. The thermally robust illumination probe of claim 3, wherein the high temperature distal part (600) has a flared proximal end (602).

6. The thermally robust illumination probe of claim 1, wherein a portion of the high temperature distal part is coated with a reflective coating (312, 512, 604) and wherein the reflective dielectric coating is a Teflon® or a multilayer dielectric stack coating.

7. The thermally robust illumination probe of claim 1, wherein the reflective metal coating (312,512) is silver.

8. The thermally robust illumination probe of claim 1, wherein the housing is made out of one or more biocompatible materials including plastic.

9. The thermally robust illumination probe of claim 1, wherein the housing (508) is configured for anchoring the thermally robust illumination probe at a fixed position during a surgery.

10. The thermally robust illumination probe of claim 1, wherein the housing (408) is configured for integrating a wide angle lens (414) at the distal end of the high temperature distal part.

11. The thermally robust illumination probe of claim 1, wherein the housing (508) is configured for accommodating a channel (516) for fluid flow, in addition to light transmission through the optically bonded plastic optical fiber (502) and the high temperature distal part (506).

12. The thermally robust illumination probe of claim 11, wherein the housing comprises a hub (510) connected to a cannula (508).

13. The thermally robust illumination probe of claim 12, wherein the distal end of the cannula (508) is beveled for entry to a surgery site through an incision.

14. The thermally robust illumination probe of claim 12, further comprising an optical adhesive bonding (504) the plastic optical fiber (502) and the high temperature distal part (506), wherein the optical adhesive is spilled over outside a plastic hub (510) to position the plastic optical fiber in a desired position.

15. The thermally robust illumination probe of claim 1, further comprising an optical adhesive (204,304,404,504) bonding the plastic optical fiber (202,302,402,502) and the high temperature distal part (206,306,406,506,600).

16. The thermally robust illumination probe of claim 15, wherein the optical adhesive provides adhesion between the plastic optical fiber, the high temperature distal part, and the housing.

17. The thermally robust illumination probe of claim 1, wherein the high temperature distal part is an omni-directional reflective hollow cannula (900) whose inner surface is coated with a reflective coating consisting of dielectric materials that create, in the visible wavelength band, a one-dimensional bandgap in the reflective coating that prevents photons from entering the cannula.

## Patentansprüche

1. Hitzfeste Beleuchtungssonde (200, 300, 400, 500), aufweisend:
eine Optikfaser (202, 302, 402, 502) aus Kunststoff;
ein Hochtemperatur-Distal-Bestandteil (206, 306, 406, 506, 600); und
ein Gehäuse (208, 310, 408, 508);
wobei die Kunststoff-Optikfaser an das Hochtemperatur-Distal-Bestandteil optisch gekoppelt ist, um Licht durch das Hochtemperatur-Distal-Bestandteil zu übertragen, wobei die Kunststoff-Optikfaser innerhalb des Gehäuses mit dem Hochtemperatur-Distal-Bestandteil zusammen verbunden ist, wobei das Hochtemperatur-Distal-Bestandteil eine optische Glasfaser mit einem Überzug (604) und einem Kern (603) ist, die der Faser das Erreichen einer numerischen Apertur von 0,66 ermöglichen, **dadurch gekennzeichnet, dass** der Kern an einem distalen Ende (601) offen liegt, und sich das distale Ende (601) in einen kegelförmigen Bestandteil eines parabolischen Konzentrators verjüngt.

2. Hitzfeste Beleuchtungssonde nach Anspruch 1, wobei das Gehäuse ein Zylinder (508) aus rostfreiem Stahl oder biologisch kompatiblem Material ist.

3. Hitzfeste Beleuchtungssonde nach Anspruch 1, wobei das Hochtemperatur-Distal-Bestandteil (600) aus einem oder mehreren Hochtemperatur-Materialen gefertigt ist.

4. Hitzfeste Beleuchtungssonde nach Anspruch 3, wobei das Hochtemperatur-Distal-Bestandteil (600) ein ausgeformtes Distalende (601) hat.

5. Hitzfeste Beleuchtungssonde nach Anspruch 3, wobei das Hochtemperatur-Distal-Bestandteil (600) ein aufgeweitetes proximales Ende (602) hat.

6. Hitzfeste Beleuchtungssonde nach Anspruch 1, wobei ein Anteil des Hochtemperatur-Distal-Bestandteils mit einer reflektiven Beschichtung (312, 512, 604) beschichtet ist, und wobei die reflektive dielektrische Beschichtung eine Teflon®- oder eine mehrschichtige dielektrische Stapelbeschichtung ist.

7. Hitzfeste Beleuchtungssonde nach Anspruch 1, wobei die reflektive Metallbeschichtung (312, 512) Silber ist.

8. Hitzfeste Beleuchtungssonde nach Anspruch 1, wobei das Gehäuse aus einem oder mehreren biologisch kompatiblen Materialen einschließlich Kunststoff gefertigt ist.

9. Hitzfeste Beleuchtungssonde nach Anspruch 1, wobei das Gehäuse (508) ausgelegt ist, um die hitzfeste Beleuchtungssonde an einer festen Position während einer Operation zu verankern.

10. Hitzfeste Beleuchtungssonde nach Anspruch 1, wobei das Gehäuse (408) ausgelegt ist, eine Weitwinkel-Optik (414) an dem distalen Ende des Hochtemperatur-Distal-Bestandteils zu integrieren.

11. Hitzfeste Beleuchtungssonde nach Anspruch 1, wobei das Gehäuse (508) ausgelegt ist, zusätzlich zu der Lichtübertragung durch die optisch verbundene Kunststoff-Optikfaser (502) mit Hochtemperatur-Distal-Bestandteil (506) einen Kanal (516) für einen Fluidstrom aufzunehmen.

12. Hitzfeste Beleuchtungssonde nach Anspruch 11, wobei das Gehäuse eine Nabe (510) aufweist, die mit einem Zylinder (508) verbunden ist.

13. Hitzfeste Beleuchtungssonde nach Anspruch 12, wobei das distale Ende des Zylinders (508) gekrümmt ist, um es an operative Stelle durch einen Einschnitt einzuführen.

14. Hitzfeste Beleuchtungssonde nach Anspruch 12, ferner aufweisend eine optische Haftverbindung (504) der Kunststoff-Optikfaser (502) mit dem Hochtemperatur-Distal-Bestandteil (506), wobei die optische Haftung über den Außenraum einer Kunststoff-Nabe (510) vergossen ist, um die Kunststoff-Optikfaser in einer gewünschten Position zu positionieren.

15. Hitzfeste Beleuchtungssonde nach Anspruch 1, ferner aufweisend eine optische Haftung (204, 304, 404,504), die die Kunststoff-Optikfaser (202, 302, 402, 502) und das Hochtemperatur-Distal-Bestandteil (206, 306, 406, 506, 600) verbindet.

16. Hitzfeste Beleuchtungssonde nach Anspruch 15, wobei die optische Haftung eine Adhäsion zwischen der Kunststoff-Optikfaser, dem Hochtemperatur-Distal-Bestandteil und dem Gehäuse schafft.

17. Hitzfeste Beleuchtungssonde nach Anspruch 1, wobei das Hochtemperatur-Distal-Bestandteil ein omnidirektionaler reflektiver Hohlzylinder (900) ist, dessen Innenfläche mit einer reflektiven Beschichtung beschichtet ist, die aus dielektrischen Materialen besteht, die in dem sichtbaren Wellenlängenband einen eindimensionalen Bandspalt in der reflektiven Beschichtung schaffen, wodurch Photonen vom Eindringen in den Zylinder gehindert sind.

## Revendications

1. Sonde d'illumination thermorésistante (200, 300, 400, 500) comprenant:
une fibre optique en plastique (202, 302, 402, 502) ;
une partie distale à haute température (206, 306, 406, 506, 600) ; et
un boîtier (208, 310, 408, 508) ;
dans laquelle la fibre optique en plastique est optiquement couplée à la partie distale à haute température pour transmettre la lumière par le biais de la partie distale à haute température, laquelle la fibre optique en plastique est reliée à la partie distale à haute température à l'intérieur du boîtier ;
dans laquelle la partie distale à haute température est une fibre optique en verre ayant une gaine optique (604) et un noyau (603) adaptés pour permettre à la fibre d'obtenir une ouverture numérique de 0,66, **caractérisée en ce que** le noyau est exposé au niveau d'une extrémité distale (601), et l'extrémité distale (601) est rétrécie dans un concentrateur parabolique composé, en forme de cône.

2. Sonde d'illumination thermorésistante selon la revendication 1, dans laquelle le boîtier est une canule (508) fabriquée à partir d'acier inoxydable ou à partir d'un matériau biocompatible.

3. Sonde d'illumination thermorésistante selon la revendication 1, dans laquelle la partie distale à haute température (600) est fabriquée avec un ou plusieurs matériaux à haute température.

4. Sonde d'illumination thermorésistante selon la revendication 3, dans laquelle la partie distale à haute température (600) a une extrémité distale sculptée (601).

5. Sonde d'illumination thermorésistante selon la revendication 3, dans laquelle la partie distale à haute température (600) a une extrémité proximale évasée (602).

6. Sonde d'illumination thermorésistante selon la revendication 1, dans laquelle une partie de la partie distale à haute température est recouverte d'un revêtement réfléchissant (312, 512, 604), et dans laquelle le revêtement diélectrique réfléchissant est un Téflon® ou un revêtement empilé diélectrique multicouche.

7. Sonde d'illumination thermorésistante selon la revendication 1, dans laquelle le revêtement métallique réfléchissant (312, 512) est de l'argent.

8. Sonde d'illumination thermorésistante selon la revendication 1, dans laquelle le boîtier est fabriqué avec un ou plusieurs matériaux biocompatibles, comprenant le plastique.

9. Sonde d'illumination thermorésistante selon la revendication 1, dans laquelle le boîtier (508) est configuré pour ancrer la sonde d'illumination thermorésistante dans une position fixe au cours d'une chirurgie.

10. Sonde d'illumination thermorésistante selon la revendication 1, dans laquelle le boîtier (408) est configuré pour intégrer un objectif grand-angle (414) au niveau de l'extrémité distale de la partie distale à haute température.

11. Sonde d'illumination thermorésistante selon la revendication 1, dans laquelle le boîtier (508) est configuré pour loger un canal (516) destiné à l'écoulement de fluide, en plus de la transmission de lumière par le biais de la fibre optique en plastique (502) reliée de manière optique et la partie distale à haute température (506).

12. Sonde d'illumination thermorésistante selon la revendication 11, dans laquelle le boîtier comprend un raccord (510) connecté à une canule (508).

13. Sonde d'illumination thermorésistante selon la revendication 12, dans laquelle l'extrémité distale de la canule (508) est biseautée pour entrer sur un site chirurgical par une incision.

14. Sonde d'illumination thermorésistante selon la revendication 12, comprenant en outre un adhésif optique (504) liant la fibre optique en plastique (502) et la partie distale à haute température (506), lequel adhésif optique se propage à l'extérieur d'un raccord en plastique (510) pour positionner la fibre optique en plastique dans une position souhaitée.

15. Sonde d'illumination thermorésistante selon la revendication 1, comprenant en outre un adhésif optique (204, 304, 404, 504) liant la fibre optique en plastique (202, 302, 402, 502) et la partie distale à haute température (206, 306, 406, 506, 600).

16. Sonde d'illumination thermorésistante selon la revendication 15, dans laquelle l'adhésif optique fournit l'adhérence entre la fibre optique en plastique, la partie distale à haute température et le boîtier.

17. Sonde d'illumination thermorésistante selon la revendication 1, dans laquelle la partie distale à haute température est une canule creuse réfléchissante omnidirectionnelle (900) dont la surface interne est recouverte d'un revêtement réfléchissant composé de matériaux diélectriques qui créent, dans la bande de longueur d'onde visible, une bande interdite unidimensionnelle dans le revêtement réfléchissant qui empêche l'entrée des photons dans la canule.
